**Europäisches Patentamt**

**European Patent Office**

**Office européen des brevets**

(19)

(11) Veröffentlichungsnummer: **0 327 917 B1**

(12) # EUROPÄISCHE PATENTSCHRIFT

(45) Veröffentlichungstag der Patentschrift:
04.12.91 Patentblatt 91/49

(51) Int. Cl.⁵: **G10K 11/00, G10K 11/34**

(21) Anmeldenummer: 89101562.0

(22) Anmeldetag: 30.01.89

(54) **Stosswellenquelle zum berührungslosen Zertrümmern von Konkrementen im Körper eines Lebewesens.**

(30) Priorität: 10.02.88 DE 3804096

(43) Veröffentlichungstag der Anmeldung:
16.08.89 Patentblatt 89/33

(45) Bekanntmachung des Hinweises auf die
Patenterteilung:
04.12.91 Patentblatt 91/49

(84) Benannte Vertragsstaaten:
DE FR GB NL

(56) Entgegenhaltungen:
DE-A- 2 657 163
DE-A- 3 119 295
GB-A- 2 140 693
US-A- 3 990 300

(73) Patentinhaber: SIEMENS
AKTIENGESELLSCHAFT
Wittelsbacherplatz 2
W-8000 München 2 (DE)

(72) Erfinder: Hassler, Dietrich, Dipl.-Ing.
Flurweg 3
W-8525 Uttenreuth (DE)

**Beschreibung**

Die Erfindung betrifft eine Stoßwellenquelle zum berührungslosen Zertrümmern von Konkrementen im Körper eines Lebewesens, welche eine Vielzahl von mosaikartig in einer konkaven Fläche angeordneten elektroakustischen Wandlern aufweist, die zur Erzeugung von Stoßwellen in einem an die Wandler angrenzenden Ausbreitungsmedium mittels einer Ansteuereinrichtung impulsartig ansteuerbar sind, wobei jeder Wandler eine akustische Achse aufweist und die Stoßwellenquelle insgesamt eine akustische Achse besitzt und wobei sich die akustischen Achsen der Wandler in einem auf der akustischen Achse der Stoßwellenquelle liegenden Fokus schneiden.

Derartige Stoßwellenquellen werden z.B. mittels einer das Ankoppelmedium einschließenden flexiblen Membran an den Körper des zu behandelnden Lebewesens angepreßt. Dabei wird mittels einer geeigneten Ortungseinrichtung sichergestellt, daß sich das zu zertrümmernde Konkrement im Fokus der Stoßwellenquelle befindet. Unter der Einwirkung der von der Stoßwellenquelle ausgesandten Stoßwellen zerfällt das Konkrement in Bruchstücke, die auf natürlichem Wege abgehen können.

Eine Stoßwellenquelle der eingangs genannten Art ist aus der DE-OS 3319871 bekannt. Dabei sind die Wandler in einer kugelkalottenförmigen Fläche angeordnet, so daß der Fokus der Stoßwellenquelle dem Krümmungsmittelpunkt dieser Fläche entspricht. Demzufolge weisen die Wandler der bekannten Stoßwellenquelle dann, wenn Konkremente zertrümmert werden sollen, die nahe bei der Körperoberfläche des Lebewesens liegen, einen vergleichsweise großen Abstand von der Körperoberfläche des Lebewesens auf. Da die bekannte Stoßwellenquelle einen konstanten Öffnungswinkel aufweist, der durch den Krümmungsradius der kugelkalottenförmigen Fläche und deren Durchmesser bestimmt ist, müssen die Stoßwellen dann über einen sehr kleinen Bereich der Körperoberfläche in den Körper des Lebewesens eingekoppelt werden. Dies führt zu einer unerwünscht hohen und unter Umständen sogar schädlichen Leistungsdichte an der Körperoberfläche des zu behandelnden Lebewesens.

Wenn die Ortung der Konkremente mittels einer im Zentrum der kugelkalottenförmigen Fläche angeordneten Ultraschall-Ortungseinrichtung erfolgen soll, ergeben sich weitere Nachteile, Wird nämlich die Ultraschall-Ortungseinrichtung so angeordnet, daß sie, was physikalisch am sinvollsten ist, unter Zwischenschaltung der Ankoppelmembran an der Körperoberfläche des zu behandelnden Lebewesens anliegt, deckt sie bei der Behandlung von nahe bei der Körperoberfläche des Lebewesens liegenden Konkrementen einen erheblichen Teil des an sich zur Einkoppelung der Stoßwellen zur Verfügung stehenden Bereiches der Körperoberfläche ab, so daß zur Sicherstellung des Behandlungserfolges die Leistungsdichte in dem zur Einkoppelung der Stoßwellen verbleibenden Teil der Körperoberfläche weiter erhöht werden muß. Wird dagegen die Ultraschall-Ortungseinrichtung in einer solchen Entfernung von der Körperoberfläche des Lebewesens angeordnet, daß ein ausreichend großer Bereich der Körperoberfläche zur Einkoppelung der Stoßwellen zur Verfügung steht, treten an der Ankoppelmembran Reflexionen der von der Ultraschall-Ortungseinrichtung ausgesandten Ultraschallwellen auf, die zu Bildartefakten führen, die die Ortung des zu zertrümmernden Konkrementes erschweren oder sogar unmöglich machen.

Außerdem ist aus der DE-OS 3119295 eine Stoßwellenquelle bekannt, bei der die Möglichkeit besteht, die Wandler mittels der Ansteuereinrichtung mit zeitlichem Versatz derart anzusteuern, daß die von den einzelnen Wandlern ausgehenden Stoßwellen gleichzeitig in dem Fokus der Stoßwellenquelle eintreffen. Bei der bekannten Stoßwellenquelle ist eine auf die Ansteuereinrichtung einwirkende Steuereinrichtung vorgesehen, mittels derer der zeitliche Versatz, mit dem die Ansteuereinrichtung die Wandler ansteuert, derart veränderbar ist, daß der Fokus der Stoßwellenquelle längs der akustischen Achse der Stoßwellenquelle verlagerbar ist. Die bekannte Stoßwellenquelle weist also einen auf elektronischem Wege veränderbaren Fokusabstand auf, d.h., die Stoßwellenquelle besitzt eine elektronische Fokussierung. Es besteht somit die Möglichkeit, im Falle von nahe bei der Körperoberfläche des Lebewesens liegenden Konkrementen die Stoßwellenquelle mit einem geringen Fokusabstand und damit einem großen Öffnungswinkel zu betreiben, so daß die Leistungsdichte an der Körperoberfläche des zu behandelnden Lebewesens in erträglichen Grenzen gehalten werden kann. Gleichzeitig kann im Falle der Verwendung einer Ultraschall-Ortungseinrichtung diese unter Zwischenfügung der Ankoppelmembran an die Körperoberfläche des zu behandelnden Lebewesens angelegt werden, ohne daß der zur Einkoppelung der Stoßwellen zur Verfügung stehende Bereich der Körperoberfläche durch die Ultraschall-Ortungseinrichtung in unzulässiger Weise verringert wird. Allerdings darf im Falle der bekannten Stoßwellenquelle die Erstreckung jedes Wandlers quer zur Richtung seiner akustischen Achse ein Achtel der Wellenlänge der von dem Wandler ausgesandten Stoßwelle nicht wesentlich überschreiten, da ansonsten zu große Laufwegunterschiede zwischen den vom Rand und den vom Zentrum des jeweiligen Wandlers abgestrahlten Anteilen der Stoßwelle zum Fokus auftreten würden, so daß eine wirksame Fokussierung unmöglich wäre. Demzufolge muß die bekannte Stoßwellenquelle eine sehr hohe Anzahl von relativ kleinen Wandlern aufweisen, um Stoßwellen ausreichender Energie und ausreichenden Fokussierungsgrades abgeben zu können. Dies zieht neben einem aufwendigen Aufbau der Stoßwellenquelle selbst eine komplizierte Ansteuereinrichtung und eine komplizierte Steuereinrichtung nach sich. Außerdem ist bei sehr kleinen Wandlern eine ausreichende elektrische Spannungsfestigkeit mit vertretbarem Aufwand nicht zu gewährleisten.

Der Erfindung liegt die Aufgabe zugrunde, eine Stoßwellenquelle der eingangs genannten Art so auszubilden, daß auch bei der Behandlung von dicht bei der Körperoberfläche des zu behandelnden Lebewesens liegenden Konkrementen eine geringe Leistungsdichte an der Körperoberfläche auftritt und dennoch ein einfacher Aufbau und eine hohe elektrische Spannungsfestigkeit der Stoßwellenquelle gewährleistet ist.

Nach der Erfindung wird diese Aufgabe dadurch gelöst, daß die Wandler derart schwenkbar angeordnet sind, daß die akustische Achse jedes Wandlers in einer die akustische Achse der Stoßwellenquelle enthaltenden Ebene verschwenkbar ist, und daß Stellmittel vorgesehen sind, mittels derer die Wandler zur Verlagerung des Fokus der Stoßwellenquelle zwischen einem näheren und einem ferneren Fokusabstand gemeinsam schwenkbar sind und die die Wandler derart ausgerichtet halten, daß sich ihre akustischen Achsen in dem Fokus der Stoßwellenquelle schneiden.

Im Falle der erfindungsgemäßen Stoßwellenquelle besteht somit die Möglichkeit, deren Fokus zwischen einem näheren und einem ferneren Fokusabstand zu verstellen, was zur Folge hat, daß auch der Öffnungswinkel der Stoßwellenquelle verstellbar ist. Es besteht somit die Möglichkeit, den Fokusabstand und damit den Öffnungswinkel der Stoßwellenquelle den Bedürfnissen des jeweiligen Behandlungsfalles entsprechend so einzustellen, daß bei der Einkoppelung der Stoßwellen in den Körper des Lebewesens nur eine geringe Leistungsdichte an der Körperoberfläche auftritt. Zugleich ist es problemlos möglich, eine Ultraschall-Ortungseinrichtung im Zentrum der Stoßwellenquelle anzuordnen. Außerdem ist ein komplizierter Aufbau der Stoßwellenquelle vermieden, da infolge des Umstandes, daß die Verstellung des Fokusabstandes mechanisch erfolgt, vergleichsweise große Wandler verwendet werden können und eine aufwendige Ansteuereinrichtung mit der zugehörigen Steuereinrichtung nicht erforderlich ist. Da vergleichsweise große Wandler verwendet werden können, weisen diese in der Regel ohne besondere Maßnahmen eine ausreichende elektrische Spannungsfestigkeit auf. Allerdings treffen die von den einzelnen Wandlern abgegebenen Stoßwellen nur dann gleichzeitig im Fokus der Stoßwellenquelle ein, wenn unter Berücksichtigung der Gestalt der konkaven Fläche derjenige Fokusabstand eingestellt ist, bei dem die Laufzeit der Stoßwellen von den einzelnen Wandlern zu dem Fokus für alle Wandler gleich ist, also der Fokusabstand eingestellt ist, bei dem alle Wandler den gleichen Abstand zum Fokus der Stoßwellenquelle besitzen. Ein zeitlich versetztes Eintreffen der von den einzelnen Wandlern ausgehenden Stoßwellen im Fokus der Stoßwellenquelle ist in der Praxis jedoch nicht unbedingt unerwünscht, da sich gezeigt hat, daß auch in diesem Falle gute Behandlungserfolge erzielbar sind.

Nach einer Variante der Erfindung ist vorgesehen, daß die Wandler mittels der Ansteuereinrichtung mit zeitlichem Versatz derart ansteuerbar sind, daß die von den einzelnen Wandlern ausgehenden Stoßwellen gleichzeitig in dem Fokus der Stoßwellenquelle eintreffen, wobei eine auf die Ansteuereinrichtung einwirkende Steuereinrichtung vorgesehen ist, mittels derer der zeitliche Versatz, mit dem die Ansteuereinrichtung die Wandler ansteuert, veränderbar ist, und wobei die Stellmittel derart auf die Steuereinrichtung einwirken, daß diese den zeitlichen Versatz an die jeweilige Schwenkstellung der Wandler anpaßt. Da neben dieser elektronischen auch eine mechanische Fokussierung vorgesehen ist, können wesentlich größere Wandlerelemente als im Falle von Stoßwellenquellen, bei denen die Fokussierung ausschließlich auf elektronischem Wege erfolgt, verwendet werden. Dies hat zur Folge, daß in vorteilhafter Weise die Ansteuereinrichtung und die zugehörige Steuereinrichtung im Vergleich zum Stand der Technik wesentlich einfacher ausgebildet sind.

Wenn gemäß einer Ausführungsform der Erfindung die Wandler jeweils fokussierte Stoßwellen abstrahlen, wobei jeder Wandler einen auf der akustischen Achse des Wandlers liegenden Fokus aufweist, der von dem Wandler einen Fokusabstand aufweist, der dem Mittelwert aus dem näheren und dem ferneren Fokusabstand der Stoßwellenquelle entspricht, ist ein Fokus mit einer sehr kleinen räumlichen Erstreckung realisierbar.

Als elektroakustische Wandler sind im Falle der Erfindung vorzugsweise piezoelektrische Wandler vorgesehen.

Eine Variante der Erfindung sieht vor, daß die Wandler zu Gruppen zusammengefaßt sind, von denen jede mehrere Wandler enthält, die in Form eines Kreisringes angeordnet sind, dessen Mittelachse der akustischen Achse der Stoßwellenquelle entspricht. Durch diese Maßnahme wird der Vorteil erzielt, daß die Laufzeit der von den Wandlern einer Gruppe ausgehenden Stoßwellen zum Fokus der Stoßwellenquelle jeweils gleich ist. Dies ermöglicht für den Fall, daß eine elektronische Fokussierung der Stoßwellenquelle vorgesehen ist, einen weiter vereinfachten Aufbau der Ansteuereinrichtung und der zugehörigen Steuereinrichtung, da dann die Wandler einer Gruppe jeweils gleichzeitig angesteuert werden können. Außerdem ermöglicht die Zusammenfassung der Wandler zu Gruppen auch einen vereinfachten Aufbau der Stellmittel, da sämtliche Wandler einer Gruppe jeweils die gleiche Schwenkstellung in bezug auf die akustische Achse der Stoßwellenquelle einnehmen und somit die Möglichkeit besteht, alle Wandler einer Gruppe gemeinsam zu schenken.

Ein einfacher konstruktiver Aufbau der erfindungsgemäßen Stoßwellenquelle ist gewährleistet, wenn gemäß einer Variante der Erfindung vorgesehen ist, daß die Wandler an einem gemeinsamen Halteteil angebracht sind, wobei jeder Wandler um eine Achse schwenkbar ist, die rechtwinklig auf der die akustische Achse des Wandlers und die akustische Achse der Stoßwellenquelle enthaltenden Ebene steht.

Eine konstruktiv einfache Ausbildung der Stellmittel wird erreicht, wenn gemäß einer Ausführungsform der Erfindung vorgesehen ist, daß die Stellmittel eine der Anzahl der Wandler entsprechende Anzahl von Hebeln, Stellglieder für die Hebel sowie Betätigungsmittel für die Stellglieder aufweisen, wobei jeder Hebel mit seinem einen Ende mit einem Wandler verbunden ist und mit seinem anderen Ende mit einem Stellglied in Eingriff steht und wobei die Stellglieder unter der Wirkung der Betätigungsmittel derart auf die Hebel einwirken, daß diese die Wandler schwenken. Sofern die Wandler zu Gruppen zusammengefaßt sind, kann eine weiter vereinfachte Ausbildung der Stellmittel dadurch erreicht werden, daß eine der Anzahl der Gruppen von Wandlern entsprechende Anzahl von Stellgliedern vorgesehen ist, wobei jeweils ein Stellglied mit den zu den Wandlern einer Gruppe gehörigen Hebeln in Eingriff steht. Eine zusätzliche konstruktive Vereinfachung der Stellmittel ist erreichbar, wenn gemeinsame Betätigungsmittel für die Stellglieder vorgesehen sind, die diese gleichzeitig in bezug auf die Hebel verstellen.

Gemäß einer weiteren Variante der Erfindung ist vorgesehen, daß im Zentrum der konkaven Fläche eine Ultraschall-Ortungseinrichtung zum Orten des zu zertrümmernden Konkrementes angeordnet ist.

Eine Ausführung der Erfindung sieht zur Steigerung der Spannungsfestigkeit der Stoßwellenquelle vor, daß zwischen dem Ausbreitungsmedium und den Wandlern eine elastisch nachgiebige Trennmembran angeordnet ist, an welcher die Wandler mit ihren die Stoßwellen abstrahlenden Flächen anliegen, und daß die Wandler von einer elektrisch isolierenden Flüssigkeit umgeben sind, die durch die Trennmembran von dem Ausbreitungsmedium getrennt ist.

Ausführungsbeispiele der Erfindung sind in den beigefügten Zeichnungen dargestellt. Es zeigen :

Fig. 1    in schematischer Darstellung einen Längsschnitt durch eine erfindungsgemäße Stoßwellenquelle,
Fig. 2    in stark vereinfacher Darstellung eine Ansicht der erfindungsgemäßen Stoßwellenquelle, und
Fig. 3    in stark schematisierter Darstellung einen Längsschnitt durch eine erfindungsgemäße Stoßwellenquelle.

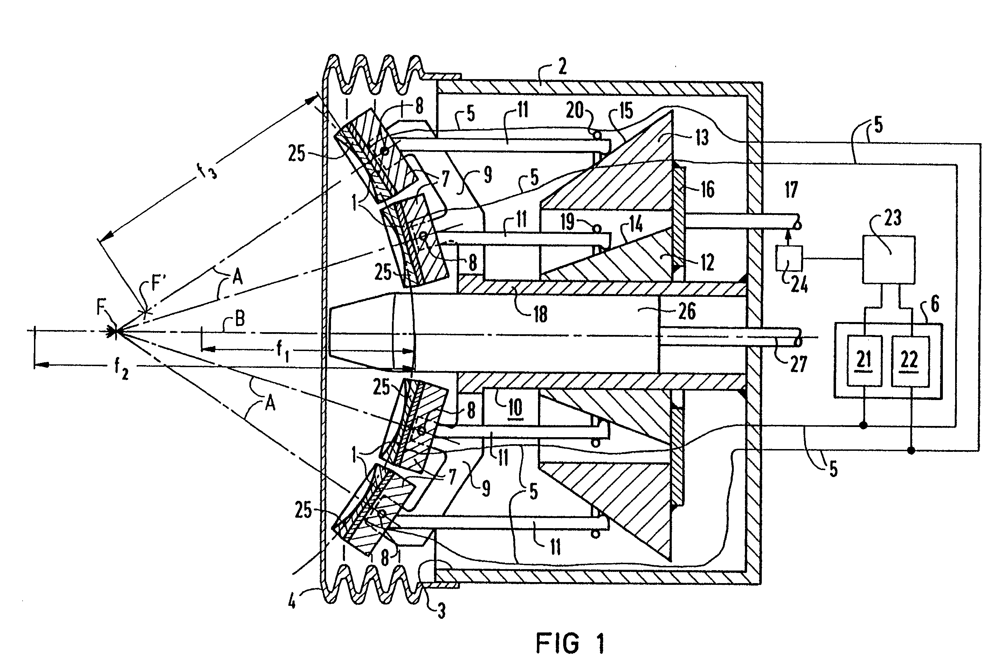

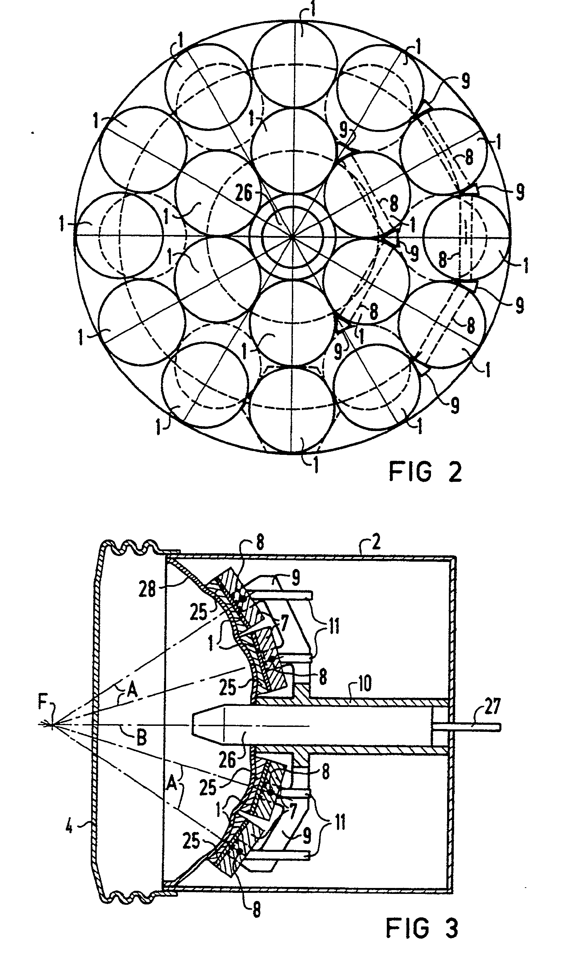

Wie aus den Figuren ersichtlich ist, weist die erfindungsgemäße Stoßwellenquelle eine Anzahl von piezoelektrischen Wandlern 1 auf, die in einer konkaven rotationssymmetrischen Fläche angeordnet sind, wobei diese Fläche in Fig. 2 der Übersichtlichkeit halber eben dargestellt ist. Es handelt sich dabei um eine kugelkalottenförmige Fläche, die in Fig. 1 strickpunktiert angedeutet ist. Die piezoelektrischen Wandler 1 sind in einem Gehäuse 2 angeordnet, das eine Austrittsöffnung 3 für von den piezoelektrischen Wandlern 1 abgegebene Stoßwellen aufweist, die mittels einer flexiblen Membran 4 verschlossen ist. Der von dem Gehäuse 2 und der Membran 4 umgrenzte Raum ist mit einer Flüssigkeit, z.B. Wasser, als Ausbreitungsmedium für die von den piezoelektrischen Wandlern 1 abgegebenen Stoßwellen gefüllt. Während der Behandlung eines Lebewesens wird die Stoßwellenquelle mit ihrer Membran 4 an den Körper des Lebewesens zur akustischen Ankoppelung angepreßt.

Jeder piezoelektrische Wandler 1 weist eine akustische Achse A auf, längs derer sich die von ihm abgegebenen Stoßwellen ausbreiten. Die von den piezoelektrischen Wandlern ausgehenden Stoßwellen laufen in einem Fokus F der Stoßwellenquelle zusammen, der auf der akustischen Achse B der Stoßwellenquelle liegt und der dem Schnittpunkt der akustischen Achsen A der piezoelektrischen Wandler 1 entspricht. Infolge der kugelkalottenförmigen Gestalt der Fläche, in der die piezoelektrischen Wandler 1 angeordnet sind, entspricht die akustische Achse B der Stoßwellenquelle der Mittelachse der kugelkalottenförmigen Fläche.

Die piezoelektrischen Wandler 1 sind über Leitungen 5 mit einer schematisch angedeuteten Ansteuereinrichtung 6 verbunden, die die piezoelektrischen Wandler 1 zur Erzeugung von Stoßwellen mit Spannungsimpulsen ansteuert.

Die piezoelektrischen Wandler 1 sind jeweils mit einem Träger 7 versehen, mittels dessen sie mit Bolzen 8 an Armen 9 eines gemeinsamen Halteteiles 10 schwenkbar angebracht sind. Das Halteteil 10 ist mit dem Gehäuse 2 verbunden. Dabei ist die Anordnung so getroffen, daß die piezoelektrischen Wandler 1 zu zwei Gruppen von piezoelektrischen Wandlern 1 zusammengefaßt sind.

Die piezoelektrischen Wandler 1 jeder Gruppe sind in der Form eines Kreisringes angeordnet, wobei die innere Gruppe sechs und die äußere Gruppe zwölf piezoelektrische Wandler 1 jeweils gleicher Abmessungen enthält und die Mittelachsen der Kreisringe der akustischen Achse B der Stoßwellenquelle entsprechen.

Jeder piezoelektrische Wandler 1 ist mit einem Hebel 11 versehen. Die Hebel 11 sind mit ihren einen Enden jeweils starr mit dem Träger 7 des zugehörigen piezoelektrischen Wandlers 1 verbunden. Mit ihren anderen Enden stehen die Hebel 11 jeweils einer Gruppe von piezoelektrischen Wandlern 1 mit einem zu der jeweiligen Gruppe von piezoelektrischen Wandlern 1 gehörigen ringförmigen Stellglied 12 bzw. 13 in Eingriff. die Stellglieder 12 und 13 weisen jeweils eine kegelige Eingriffsfläche 14 bzw. 15 für die anderen Enden der Hebel 11 auf, wobei die Mittelachsen der Eingriffsflächen 14 und 15 der akustischen Achse B der Stoßwellenquelle entsprechen. Die Stellglieder 12 und 13 sind mittels eines Koppelteiles 16 starr miteinander verbunden. An diesem ist eine Stange 17 angebracht, die flüssigkeitsdicht und längsverschieblich durch die Wand des Gehäuses 2 nach außen geführt ist und deren Längsachse parallel zur akustischen Achse B der Stoßwellenquelle verläuft. Das innere Stellglied 12 ist mit seiner Bohrung auf einem rohrförmigen Ansatz 18 des Halteteiles 10 längsverschiebbar geführt. Durch Betätigen der Stange 17 können somit die Stellglieder 12 und 13 in Richtung der akustischen Achse B der Stoßwellenquelle verschoben werden. Dabei wirken die Eingriffsflächen 14 und 15 der Stellglieder 12 und 13 mit den anderen Enden der Hebel 11, die übrigens durch ringförmige Gummifedern 19 bzw. 20 mit den Eingriffsflächen 14 bzw. 15 in Eingriff gehalten sind, derart zusammen, daß die piezoelektrischen Wandler 1 geschwenkt werden.

Die piezoelektrischen Wandler 1 sind jeweils mit Hilfe des entsprechenden Bolzens 8 um eine Achse schwenkbar gelagert, die rechtwinklig auf einer Ebene steht, die die akustische Achse A des jeweiligen piezoelektrischen Wandlers 1 und die akustische Achse B der Stoßwellenquelle enthält. Die akustische Achse A jedes piezoelektrischen Wandlers 1 ist somit in einer Ebene verschwenkbar, die die akustische Achse B der Stoßwellenquelle enthält. Gemäß Fig. 1 schneiden die Achsen, um die die piezoelektrischen Wandler 1 schwenkbar sind, jeweils die akustische Achse A des entsprechenden piezoelektrischen Wandlers 1. Die Hebel 11 sind jeweils an dem zugehörigen piezoelektrischen Wandler 1 derart angebracht, daß sich dann, wenn die Hebel 11 mit den Eingriffsflächen 14 bzw. 15 in Eingriff stehen, die akustischen Achsen A aller piezoelektrischen Wandler 1 wie bereits erwähnt in dem Fokus F der Stoßwellenquelle schneiden. Die Kegelwinkel der Eingriffsflächen 14 und 15 der Stellglieder 12 und 13 sind derart bemessen, daß sich für jede Stellung der Stellglieder 12 und 13, in die diese durch Betätigen der Stange 17 gebracht werden können, die akustischen Achsen A der piezoelektrischen Wandler 1 in einem auf der akustischen Achse B der Stoßwellenquelle liegenden Fokus F schneiden. Dabei ist der Fokus F zwischen einem näheren Fokusabstand $f_1$ und einem ferneren Fokusabstand $f_2$ der Stoßwellenquelle stufenlos längs der akustischen Achse B der Stoßwellenquelle verstellbar.

Die Ansteuereinrichtung 6 weist zwei Ansteuereinheiten 21 und 22 auf, von denen die Ansteuereinheit 21 die piezoelektrischen Wandler 1 der inneren Gruppe und die Ansteuereinheit 22 die der äußeren Gruppe ansteuert. Da die von den piezoelektrischen Wandlern 1 der inneren bzw. der äußeren Gruppe ausgehenden Stoßwellen im Normalfall in Abhängigkeit von dem jeweils eingestellten Fokusabstand unterschiedlich lange Wege zum jeweils eingestellten Fokus F der Stoßwellenquelle zurückzulegen haben, ist eine Steuereinrichtung 23 vorgesehen, die mit den Ansteuereinheiten 21 und 22 verbunden ist und der das Ausgangssignal eines mit der Stange 17 verbundenen schematisch angedeuteten Weggebers 24 zugeführt ist. Das Ausgangssignal des Weggebers 24 stellt ein Maß für den jeweils eingestellten Fokusabstand dar, anhand dessen die Steuereinrichtung 23 die Ansteuereinheiten 21 und 22 mit einem solchen zeitlichen Versatz betätigt, daß die von den piezoelektrischen Wandlern 1 der beiden Gruppen ausgehenden Stoßwellen gleichzeitig am jeweils eingestellten Fokus F der Stoßwellenquelle eintreffen. Dabei sind infolge des Umstandes, daß die piezoelektrischen Wandler 1 einer Gruppe jeweils den gleichen Abstand vom Fokus F der Stoßwellenquelle aufweisen nur die beiden Ansteuereinheiten 21 und 22 erforderlich.

Wie aus der Fig. 1 ersichtlich ist, ist jeder piezoelektrische Wandler 1 mit einer akustischen Sammellinse 25 versehen, so daß er fokussierte Stoßwellen aussendet. Die akustischen Sammellinsen 25 sämtlicher piezoelektrischer Wandler weisen den gleichen Fokusabstand $f_3$ auf, der so bemessen ist, daß er dem Mittelwert aus dem näheren und dem ferneren Fokusabstand $f_1$ bzw. $f_2$ der Stoßwellenquelle entspricht. Dies ist in Fig. 1 dadurch verdeutlicht, daß für einen piezoelektrischen Wandler 1 der Fokus F' und der Fokusabstand $f_3$ eingetragen sind. Infolge des Umstandes, daß die einzelenen piezoelektrischen Wandler 1 fokussierte Stoßwellen aussenden, weist die Stoßwellenquelle eine räumlich eng begrenzte Fokuszone auf.

Wie aus der Fig. 2 ersichtlich ist, weisen die einzelnen piezoelektrischen Wandler 1 eine kreisscheibenförmige Gestalt auf. Wie am Beispiel eines piezoelektrischen Wandlers 1 strichliert angedeutet ist, können diese jedoch auch von sechseckiger Gestalt sein, wobei kann bei gleicher Fläche der Kugelkalotte eine größere strahlende Fläche zur Verfügung steht. Außerdem besteht dann, wenn die akustische Achse A jedes zweiten piezoelektrischen Wandlers 1 der äußeren Gruppe wie in den Figuren dargestellt in der gleichen Ebene liegt, wie die akustische Achse A des ihm benachbarten piezoelektrischen Wandlers 1 der inneren Gruppe, die Möglichkeit, die übrigen piezoelektrischen Wandler der äußeren Gruppe radial einwärts zu versetzen, wie dies in Fig. 2 strichliert angedeutet ist. Die radial einwärts versetzten piezoelektrischen Wandler 1 bilden dann eine dritte Gruppe, die mittels einer zusätzlichen nicht dargestellten Ansteuereinheit der Ansteuereinrichtung 6 mit dem erforderlichen zeitlichen Versatz angesteuert werden muß.

Im Zentrum der kugelkalottenförmigen Fläche ist eine Ultraschall-Ortungseinrichtung 26 zum Orten des zu zertrümmernden Konkrementes angeordnet, mit deren Hilfe der Fokus F der Stoßwellenquelle auf das Konkrement ausgerichtet wird. Die Ultraschall-Ortungseinrichtung 26 ist in dem rohrförmigen Ansatz 18 des Halteteiles 10 aufgenommen und erstreckt sich somit längs der akustischen Achse B der Stoßwellenquelle. Die Ultraschall-Ortungseinrichtung 26 ist in der Bohrung des rohrförmigen Ansatzes 18 längsverschieblich, so daß sie nach Ankoppelung der Stoßwellenquelle an den Körper des zu behandelnden Lebewesens in eine solche Position gebracht werden kann, daß sie unter Zwischenschaltung der Membran 4 unmittelbar an der Körperoberfläche des Lebewesens anliegt. Zum Verschieben der Ultraschall-Ortungseinrichtung 26 ist ein Schieber 27 an dieser angebracht, der flüssigkeitsdicht und längsverschieblich durch das Gehäuse 2 nach außen geführt ist. In dem Schieber 27 können in nicht dargestellter Weise Leitungen verlaufen, über die die Ultraschall-Ortungseinrichtung 26 mit einer zu ihrem Betrieb erforderlichen elektronischen Einrichtung verbunden ist.

Darüber hinaus besteht nach erfolgter Ausrichtung des Fokus F der Stoßwellenquelle mittels der Ultraschall-Ortungseinrichtung 26 die Möglichkeit, unter Zuhilfenahme einer geeigneten elektronischen Einrichtung die an dem zu zertrümmernden Konkrement reflektierten Anteile der von der Stoßwellenquelle ausgehenden Stoßwellen mittels der piezoelektrischen Wandler 1 zu empfangen und hinsichtlich ihrer Amplitude auszuwerten. Der Fokusabstand der Stoßwellenquelle kann dann durch Betätigen der Stange 17 so eingestellt werden, daß die reflektierten Anteile der Stoßwelle eine maximale Amplitude aufweisen, was bedeutet, daß der Fokusabstand für den vorliegenden Behandlungsfall optimal eingestellt ist. Bei dieser Optimierung der Einstellung des Fokusabstandes können Stoßwellen verminderter Amplitude von der Stoßwellenquelle abgestrahlt werden.

In der Fig. 3, in der jeweils gleiche Teile mit den gleichen Bezugsziffern wie in den Fig. 1 und 2 versehen sind, ist eine erfindungsgemäße Stoßwellenquelle schematisch dargestellt, die sich von der zuvor beschriebenen dadurch unterscheidet, daß zwischen der Membran 4 und den piezoelektrischen Wandlern 1 eine ringförmige, elastisch nachgiebige Trennmembran 28 angeordnet ist, die mit ihrem äußeren Rand an der Innenwand des Gehäuses 2 und mit ihrem inneren Rand an dem rohrförmigen Ansatz 18 des Halteteiles 10 flüssigkeitsdicht, z.B. durch Kleben, befestigt ist. Der von dem Gehäuse 2 und der Membran 4 umgrenzte Raum ist somit mittels der Trennmembran 28 und des rohrförmigen Ansatzes 18 des Halteteiles 10 in zwei Teilräume unterteilt. Während der zwischen der Membran 4 und der Trennmembran 28 befindliche Raum mit einer Flüssigkeit, z.B. Wasser, als Ausbreitungsmedium für die Stoßwellen gefüllt ist, enthält der andere Teilraum, in dem sich die piezoelektrischen Wandler 1 befinden, eine elektrisch isolierende Flüssigkeit, z.B. Isolieröl, so daß eine hohe elektrische Spannungsfestigkeit der Stoßwellenquelle gewährleistet ist.

Um eine mit möglichst geringen Verlusten behaftete Einleitung der von den piezoelektrischen Wandlern 1 ausgehenden Stoßwellen über die Trennmembran 28 in das Ausbreitungsmedium zu gewährleisten, liegen die piezoelektrischen Wandler 1 jeweils mit ihrer die Stoßwellen abstrahlenden Fläche, im Falle der Fig. 3 handelt es sich dabei um die dem Fokus F der Stoßwellenquelle zugewandten Stirnflächen der akustischen Sammellinsen 25, satt an der Trennmembran 28 an. Um sicherzustellen, daß auch beim Schwenken der piezoelektrischen Wandler 1 keine Zwischenräume zwischen der Trennmembran 28 und den die Stoßwellen abstrahlenden Flächen der piezoelektrischen Wandler 1 auftreten, sind die die Stoßwellen abstrahlenden Flächen der piezoelektrischen Wandler 1 mit der Trennmembran 28 verklebt. Sofern die Trennmembran 28 die erforderlichen Elastizitätseigenschaften sowie eine geeignete Formgebung aufweist, kann eine Verklebung auch entfallen. Außerdem besteht die Möglichkeit, die Anlage der Trennmembran 28 an den piezoelektrischen Wandlern dadurch sicherzustellen, daß in dem das Ausbreitungsmedium enthaltenden Teilraum ein höherer Flüssigkeitsdruck als in dem die piezoelektrischen Wandler 1 aufnehmenden Teilraum erzeugt wird.

Um akustische Verluste zu vermeiden, ist die Trennmembran 28 aus einem Werkstoff gebildet, dessen akustische Impedanz im wesentlichen der des Ausbreitungsmediums entspricht. Sofern als Ausbreitungsmedium Wasser Verwendung findet, stellt EPDM-Gummi einen geeigneten Werkstoff für die Trennmembran 28 dar.

Hinsichtlich der in Fig. 3 nicht dargestellten Einzelheiten entspricht die Stoßwellenquelle nach Fig. 3 dem zuvor beschriebenen Ausführungsbeispiel.

## Patentansprüche

1. Stoßwellenquelle zum berührungslosen Zertrümmern von Konkrementen im Körper eines Lebewesens, welche eine Vielzahl von mosaikartig in einer konkaven Fläche angeordneten elektroakustischen Wandlern (1) aufweist, die zur Erzeugung von Stoßwellen in einem an die Wandler (1) angrenzenden Ausbreitungsmedium mittels einer Ansteuereinrichtung (6) impulsartig ansteuerbar sind, wobei jeder Wandler (1) eine akustische Achse (A) aufweist und die Stoßwellenquelle insgesamt eine akustische Achse (B) besitzt und wobei sich die akustischen Achsen (A) der Wandler (1) in einem auf der akustischen Achse (B) der Stoßwellenquelle liegenden Fokus (F) schneiden, **dadurch gekennzeichnet,** daß die Wandler (1) derart schwenkbar angeordnet sind, daß die akustische Achse (A) jedes Wandlers (1) in einer die akustische Achse (B) der Stoßwellenquelle enthaltenden Ebene verschwenkbar ist, und daß Stellmittel (11, 12, 13, 14, 15, 16, 17) vorgesehen sind, mittels derer die Wandler (1) zur Verlagerung des Fokus (F) der Stoßwellenquelle zwischen einem näheren und einem ferneren Fokusabstand ($f_1$ bzw. $f_2$) gemeinsam schwenkbar sind und die die Wandler (1) derart ausgerichtet halten, daß sich ihre akustischen Achsen (A) in dem Fokus (F) der Stoßwellenquelle schneiden.

2. Stoßwellenquelle nach Anspruch 1, **dadurch gekennzeichnet,** daß die Wandler (1) mittels der Ansteuereinrichtung (6) mit zeitlichem Versatz derart ansteuerbar sind, daß die von den einzelnen Wandlern (1) ausgehenden Stoßwellen gleichzeitig in dem Fokus (F) der Stoßwellenquelle eintreffen, wobei eine auf die Ansteuereinrichtung (6) einwirkende Steuereinrichtung (23) vorgesehen ist, mittels derer der zeitliche Versatz, mit dem die Ansteuereinrichtung (6) die Wandler (1) ansteuert, veränderbar ist, und wobei die Stellmittel (11, 12, 13, 14, 15, 16, 17, 18) derart auf die Steuereinrichtung (23) einwirken, daß diese den zeitlichen Versatz an die jeweilige Schwenkstellung der Wandler (1) anpaßt.

3. Stoßwellenquelle nach Anspruch 1 oder 2, **dadurch gekennzeichnet,** daß die Wandler (1) jeweils fokussierte Stoßwellen abstrahlen, wobei jeder Wandler (1) einen auf der akustischen Achse (A) des Wandlers (1) liegenden Fokus (F') aufweist, der von dem Wandler (1) einen Fokusabstand ($f_3$) aufweist, der dem Mittelwert aus dem näheren und dem ferneren Fokusabstand ($f_1$ bzw. $f_2$) der Stoßwellenquelle entspricht.

4. Stoßwellenquelle nach einem der Ansprüche 1 bis 3, **dadurch gekennzeichnet,** daß als elektroakustische Wandler piezoelektrische Wandler (1) vorgesehen sind.

5. Stoßwellenquelle nach einem der Ansprüche 1 bis 4, **dadurch gekennzeichnet,** daß die Wandler (1) zu Gruppen zusammengefaßt sind, von denen jede mehrere Wandler (1) enthält, die in Form eines Kreisringes angeordnet sind, dessen Mittelachse der akustischen Achse (B) der Stoßwellenquelle entspricht.

6. Stoßwellenquelle nach einem der Ansprüche 1 bis 5, **dadurch gekennzeichnet,** daß die Wandler (1) an einem gemeinsamen Halteteil (10) angebracht sind, wobei jeder Wandler (1) um eine Achse schwenkbar ist, die rechtwinklig auf der die akustische Achse (A) des Wandlers (1) und die akustische Achse (B) der Stoßwellenquelle enthaltenden Ebene steht.

7. Stoßwellenquelle nach einem der Ansprüche 1 bis 6, **dadurch gekennzeichnet,** daß die Stellmittel eine der Anzahl der Wandler (1) entsprechende Anzahl von Hebeln (11), Stellglieder (12, 13) für die Hebel (11) sowie Betätigungsmittel (16, 17) für die Stellglieder (12, 13) aufweisen, wobei jeder Hebel (11) mit seinem einen Ende mit einem Wandler (1) verbunden ist und mit seinem anderen Ende mit einem Stellglied (12, 13) in Eingriff steht und wobei die Stellglieder (12, 13) unter der Wirkung der Betätigungsmittel (16, 17) derart auf die Hebel (11) einwirken, daß diese die Wandler (1) schwenken.

8. Stoßwellenquelle nach Anspruch 5 oder 6 und 7, **dadurch gekennzeichnet,** daß eine der Anzahl der Gruppen von Wandlern (1) entsprechende Anzahl von Stellgliedern (12, 13) vorgesehen ist, wobei jeweils ein Stellglied (12, 13) mit den zu den Wandlern (1) einer Gruppe gehörigen Hebeln (11) in Eingriff steht.

9. Stoßwellenquelle nach Anspruch 7 oder 8, **dadurch gekennzeichnet,** daß gemeinsame Betätigungsmittel (16, 17) für die Stellglieder (12, 13) vorgesehen sind, die diese gleichzeitig in bezug auf die Hebel (11) verstellen.

10. Stoßwellenquelle nach einem der Ansprüche 1 bis 9, **dadurch gekennzeichnet,** daß im Zentrum der konkaven Fläche eine Ultraschall-Ortungseinrichtung (26) zum Orten des zu zertrümmernden Konkrementes angeordnet ist.

11. Stoßwellenquelle nach einem der Ansprüche 1 bis 10, **dadurch gekennzeichnet,** daß zwischem dem Ausbreitungsmedium und den Wandlern (1) eine elastisch nachgiebige Trennmembran (28) angeordnet ist, an welcher die Wandler (1) mit ihren die Stoßwellen abstrahlenden Flächen anliegen, und daß die Wandler von einer elektrisch isolierenden Flüssigkeit umgeben sind, die durch die Trennmembran (28) von dem Ausbreitungsmedium getrennt ist.

## Claims

1. A shock wave source for contact-free disintegration of concretions in the body of an organism, which has a plurality of electro-acoustic transducers (1) arranged on a concave surface in a mosaic-like manner, which are controllable in a pulsed fashion, by means of a control device (6), for generating shock waves in a propagation medium adjacent to the transducers (1), wherein each transducer (1) has an acoustic axis (A) and the shock wave source has in total an acoustic axis (B) and wherein the acoustic axes (A) of the transducers (1) intersect at a focal point lying on the acoustic axis (B) of the shock wave source, characterised in that the transducers (1) are pivotably arranged such that the acoustic axis (A) of each transducer (1) is able to be swivelled in a plane containing the acoustic axis (B) of the shock wave source, and in that adjusting means (11, 12, 13, 14, 15, 16, 17) are provided, by means of which the transducers (1) are able to swivel together for displacing the focal point (F) of the shock wave source between a closer and a further focal distance ($f_1$ or $f_2$) and which keep the transducers (1) aligned in such a way that their acoustic axes (A) intersect in the focal point (F) of the shock wave source.

2. A shock wave source according to claim 1, characterised in that the transducers (1) are controllable by means of the control device (6) with temporal offset in such a way that the shock waves emanating from the individual transducers arrive at the same time at the focal point (F) of the shock wave source, wherein there is provided a controlling system (23) acting on the control device (6), by means of which the temporal offset with which the control device (6) controls the transducers (1) can be altered and wherein the adjusting means (11, 12, 13, 14, 15, 16, 17, 18) act in such a manner on the controlling system (23) that it adapts the temporal offset to the respective pivotal adjustment of the transducers (1).

3. A shock wave source according to claim 1 or 2, characterised in that the transducers (1) emit in each case focused shock waves, wherein each transducer (1) has a focal point (F'), lying on the acoustic axis (A) of the transducer (1), which has a focal distance ($f_3$) from the transducer (1) corresponding to the average value of the closer and further focal point distance ($f_1$ or $f_2$) of the shock wave source.

4. A shock wave source according to one of claims 1 to 3, characterised in that piezoelectric transducers (1) are provided as electro-acoustic transducers.

5. A shock wave source according to one of claims 1 to 4, characterised in that the transducers (1) are arranged in groups, each of which contains several transducers (1) arranged in the form of a circular ring, the central axis of which corresponds to the acoustic axis (B) the shock wave source.

6. A shock wave source according to one of claims 1 to 5, characterised in that the transducers (1) are attached to a common holding part (10), wherein each transducer (1) is pivotable about an axis which is perpendicular to the plane containing the acoustic axis (A) of the transducer (1) and the acoustic axis (B) of the shock wave source.

7. A shock wave source according to one of claims 1 to 6, characterised in that the adjusting means have a number of levers (11) corresponding to the number of transducers (1), adjusting members (12, 13) for the levers (11) as well as actuating means (16, 17) for the adjusting members (12, 13), wherein each lever (11) is connected with its one end to a transducer (1) and with its other end is engaged with an adjusting member (12, 13) and wherein the adjusting members (12, 13), under the action of the actuating means (16, 17), act on the levers (11) so that they swivel the transducers (1).

8. A shock wave source according to claim 5 or 6 and 7, characterised in that a number of adjusting members (12, 13) are provided corresponding to the number of groups of transducers, wherein in each case one adjusting member (12, 13) is engaged with the levers (11) belonging to the transducers (1) of one group.

9. A shock wave source according to claim 7 or 8, characterised in that common actuating means (16, 17) are provided for the adjusting members (12, 13), which adjust them at the same time in relation to the levers (11).

10. A shock wave source according to one of claims 1 to 9, characterised in that arranged in the centre of the concave surface there is an ultrasonic locating device (26) for locating the concretion to be disintegrated.

11. A shock wave according to one of claims 1 to 10, characterised in that between the propagation medium and the transducers (1) there is arranged an elastically flexible separating membrane (28), on which the transducers (1) rest with their faces emitting the shock waves and in that the transducers are surrounded by an electrically insulating liquid, which is separated from the propagation medium by the separating membrane (28).

## Revendications

1. Générateur d'ondes de choc pour désintégrer à distance des concrétions dans le corps d'un être vivant, et qui comporte une multiplicité de transducteurs électroacoustiques (1), qui sont disposés sous la forme d'une mosaïque dans une surface concave et peuvent être commandés de façon impulsionnelle pour la production d'ondes de choc dans un milieu de propagation jouxtant les transducteurs (1), au moyen d'un dispositif de commande (6), et dans lequel chaque transducteur (1) possède un axe acoustique (A) et le générateur d'ondes de choc possède globalement un axe acoustique (B), et dans lequel les axes acoustiques (A) des transducteurs (1) se recoupent en un foyer (F) situé sur l'axe acoustique (B) du générateur d'ondes de choc, caractérisé car le fait que les transducteurs (1) sont montés de manière à pouvoir pivoter de telle sorte que l'axe acoustique (A) de chaque transducteur (1) peut pivoter dans un plan contenant l'axe acoustique (B) du générateur d'ondes de choc, et qu'il est prévu des moyens de réglage (11, 12, 13, 14, 15, 16, 17), qui permettent de faire pivoter en commun les transducteurs (1) pour déplacer le foyer (F) du générateur d'ondes de choc entre une position correspondant à une distance focale plus courte ($f_1$) et une position correspondant à une distance focale plus longue ($f_2$), et qui maintiennent les transducteurs (1) alignés de telle sorte que leurs axes acoustiques (A) se recoupent au niveau du foyer (F) du générateur d'ondes de choc.

2. Générateur d'ondes de choc selon la revendication 1, caractérisé en ce que les transducteurs (1) peuvent être commandés au moyen du dispositif de commande (6), avec un décalage temporel, de telle sorte que les ondes de choc partant des différents transducteurs (1) atteignent simultanément le foyer (F) du générateur d'ondes de choc, qu'il est prévu un appareil de commande (23), qui agit sur le dispositif de commande (6) et à l'aide duquel le décalage temporel, au moyen duquel le dispositif de commande (6) commande les transducteurs (1), peut être modifié, et que les moyens de réglage (11, 12, 13, 14, 15, 16, 17, 18) agissent sur le dispositif de commande (23) de telle sorte que ce dernier adapte le décalage temporel à la position pivotée respective des transducteurs (1).

3. Générateur d'ondes de choc suivant la revendication 1 ou 2, caractérisé par le fait que les transducteurs (1) émettent respectivement des ondes de choc focalisées, chaque transducteur (1) possédant un foyer (F') qui est situé sur l'axe acoustique (A) du transducteur (1) et qui est séparé du transducteur (1) par une distance ($f_3$) qui correspond à la valeur moyenne entre la distance focale la plus courte ($f_1$) et la distance focale la plus longue ($f_2$) du générateur d'ondes de choc.

4. Générateur d'ondes de choc suivant l'une des revendications 1 à 3, caractérisé par le fait que des transducteurs piézoélectriques (1) sont prévus en tant que transducteurs électro-acoustiques.

5. Générateur d'ondes de choc suivant l'une des revendications 1 à 4, caractérisé par le fait que les transducteurs (1) sont réunis en groupes, dont chacun contient plusieurs transducteurs (1), qui sont disposés sous la forme d'un anneau circulaire, dont l'axe central correspond à l'axe acoustique (B) du générateur d'ondes de choc.

6. Générateur d'ondes de choc suivant l'une des revendications 1 à 5, caractérisé par le fait que les transducteurs (1) sont disposés sur un élément de support commun (10), chaque transducteur (1) pouvant pivoter autour d'un axe qui est perpendiculaire au plan contenant l'axe acoustique (A) du transducteur (1) et l'axe acoustique (B) du générateur d'ondes de choc.

7. Générateur d'ondes de choc suivant l'une des revendications 1 à 6, caractérisé par le fait que les moyens de réglage contiennent un nombre, qui correspond au nombre des transducteurs (1), de leviers (11), organes de réglage (12, 13) pour les leviers (11) et moyens d'actionnement (16, 17) pour les organes de réglage (12, 13), chaque levier (11) étant raccordé par l'une de ses extrémités d'un transducteur (1) et étant en prise, par son autre extrémité, avec un organe de réglage (12, 13), tandis que les organes de réglage (12, 13) agissent sur les leviers (11) sous l'effet des moyens d'actionnement (16, 17), de manière que ces leviers fassent pivoter les transducteurs (1).

8. Générateur d'ondes de choc suivant les revendications 5 ou 6 et 7, caractérisé par le fait qu'il est prévu un nombre d'organes de réglage (12, 13), qui correspond au nombre des groupes de transducteurs (1), un organe de réglage (12, 13) respectif engrenant avec les leviers (11) associés aux transducteurs (1) d'un groupe.

9. Générateur d'ondes de choc selon la revendication 7 ou 8, caractérisé en ce qu'il est prévu, pour les organes de réglage (12, 13), des moyens communs d'actionnement (16, 17), qui déplacent simultanément ces organes par rapport aux leviers (11).

10. Générateur d'ondes de choc suivant l'une des revendications 1 à 9, caractérisé par le fait qu'un dispositif de repérage ultrasonore (26) servant à localiser la concrétion à désintégrer est disposé au centre de la surface concave.

11. Générateur d'ondes de choc suivant l'une des revendications 1 à 10, caractérisé par le fait qu'entre le milieu de propagation et les transducteurs (1) est disposée une membrane de séparation (28) flexible élastiquement, contre laquelle les transducteurs (1) s'appliquent par leurs surfaces émettant les ondes de choc, et que les transducteurs sont entourés par un liquide électriquement isolant, qui est séparé du milieu de propagation par la membrane de séparation (28).

**FIG 1**

EP 0 327 917 B1

FIG 2

FIG 3